# EUROPEAN PATENT APPLICATION

(11) **EP 3 275 367 A1**
(43) Date of publication of application: **31.01.2018**
(21) Application number: 17182182.0
(22) Date of filing: 19.07.2017
(51) Int. Cl.: A61B 5/05

(54) **METHODS FOR CALIBRATING MICROWAVE IMAGING SYSTEMS**

(30) Priority: 26.07.2016 US 201615219650
(71) Applicant: Keysight Technologies Inc., Santa Rosa, CA 95403 (US)
(72) Inventor: KASPER, Manuel, Loveland CO Colorado 80537 (US)
(74) Representative: Zimmermann, Tankred Klaus

(57) **Abstract**

Calibration methods for microwave imaging (MI) systems are disclosed. According to an aspect, an MI system has a plurality of Vector Network Analyzer (VNA) ports operatively connected to a plurality of antennas. A multiple state calibration network having predetermined parameters is operatively connected between a first VNA port of the plurality of VNA ports and a first antenna of the plurality of antennas. A method of calibrating the MI system includes determining first, second, and third pluralities of reflection coefficients associated with the plurality of VNA ports using first, second, and third calibration scenarios; removing a measurement effect of the multiple calibration network from the first, second and third pluralities of reflection coefficients; and determining error parameters for each VNA port using the first, second, and third pluralities of reflection coefficients.

## Description

### TECHNICAL FIELD

The present subject matter relates generally to imaging systems. More specifically, the present subject matter relates to methods for calibrating microwave imaging systems.

### BACKGROUND

Advances in microwave imaging have enabled commercial development of microwave imaging (MI) systems that are capable of generating two-dimensional 2-D and three-dimensional 3-D images from within test subjects including objects, animals, and humans. MI systems can have up to 160 antennas arranged in a fixed pattern around the test subject. One or more vector network analyzers (VNAs) in combination with microwave switches and interconnection cables are used to measure the reflection coefficients on all antennas and transmission path coefficients between all combinations of the antennas. Based on the measured data, reconstruction mathematics are used to create an image of the test subject. To achieve image accuracy, every VNA port (i.e. antenna connection) must be routinely and properly calibrated.

MI systems are currently calibrated by connecting known calibration standards to the VNA ports and performing well known calibration procedures including the short-open-load thru (SOLT) calibration and the short-open-load-reciprocal-thru (SOLR) calibration. During calibration all antennas are disconnected and the calibration standards are connected. After acquiring all measured calibration data the calibration standards are disconnected and the antennas are reconnected. This process is time consuming and labor intensive based on the number of antennas used. The process also requires a trained technician to perform the steps and can be prone to error if not performed correctly. As such, these calibration procedures are not practical for MI systems used on a daily basis such as hospitals and clinics.

Therefore, there is a need for improved MI system calibration techniques that require less time, fewer changes in the MI system configuration, and fewer steps to be performed by an operator.

### SUMMARY

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter.

Methods for improved calibration of MI systems having multiple VNA ports are disclosed herein. In a representative embodiment, an MI system includes multiple VNA ports operatively connected to multiple antennas. A multiple state calibration network having predetermined parameters is operatively connected between a first VNA port of the VNA ports and a first antenna of the antennas. A method of calibrating the MI system includes determining a first set of reflection coefficients associated with the VNA ports using a first calibration scenario, determining a second set of reflection coefficients associated with the VNA ports using a second calibration scenario, determining a third set of reflection coefficients associated with the VNA ports using a third calibration scenario, removing a measurement effect of the multiple state calibration network from the first, second and third reflection coefficients, and determining error parameters for each VNA port using the first, second, and third pluralities of reflection coefficients.

In other embodiments, the method of calibration may include determining a transmission path coefficients for each VNA port of the VNA ports, wherein the transmission path coefficients comprise transmission path coefficients associated with each of the remaining VNA ports.

In other embodiments, the method of calibration may include prompting an operator of the MI system to position the first and second homogenous phantoms. The second calibration scenario may include positioning a first homogenous phantom approximately equidistant from each antenna. A relative permittivity of the first homogenous phantom may be at least 2.0. The third calibration scenario may include positioning a second homogenous phantom approximately equidistant from each antenna. A relative permittivity of the second homogenous phantom may be at least two times greater than the relative permittivity of the first homogenous phantom.

In other embodiments, the VNA ports may include at least three VNA ports. The error parameters for each VNA port may each include a two port S-parameter matrix. The calibration network may be a multiple state, two port passive network and the predetermined parameters may include a two port S-parameter matrix.

In another representative embodiment, a method of calibrating the MI system includes determining a set of reflection coefficients on a VNA port connection of the multiple state calibration network, de-embedding the multiple state calibration network to determine a reflection coefficient of an antenna connection of the multiple state calibration network, determining a first set of reflection coefficients associated with the VNA ports using a first calibration scenario, determining a second set of reflection coefficients associated with the VNA ports using a second calibration scenario, determining a third set of reflection coefficients associated with the VNA ports using a third calibration scenario, removing a measurement effect of the multiple state calibration network from the first, second and third reflection coefficients, and determining error parameters for each VNA port using the first, second, and third pluralities of reflection coefficients.

In other embodiments, the method of calibration may include determining transmission path coefficients for each VNA port of the VNA ports using a short-open-load-reciprocal thru (SOLR) method. In other embodiments the multiple state calibration network may include at least three different predetermined reflective states and at least one predetermined thru state. In other embodiments the states of the multiple state calibration network are remotely selected by solid state switches.

### BRIEF DESCRIPTION OF THE DRAWINGS

The illustrated embodiments of the disclosed subject matter will be best understood by reference to the drawings, wherein like parts are designated by like numerals throughout. The following description is intended only by way of example, and simply illustrates certain selected embodiments of devices, systems, and processes that are consistent with the disclosed subject matter as claimed herein.
FIG. 1 is a block diagram of an example MI system having VNA ports and configured with a test subject in accordance with embodiments of the present disclosure.
FIG. 2 is a flow chart of an example method of calibrating the MI system in accordance with a representative embodiment of the present disclosure.
FIG. 3 is a flow chart of an example method of calibrating the MI system in accordance with another representative embodiment of the present disclosure.
FIG. 4 is a block diagram of VNA ports and antennas configured in a calibration scenario with a homogenous phantom in accordance with embodiments of the present disclosure.
FIG. 5 is an enlarged diagram of a VNA port and and an antenna of the MI system configured for measuring a reflection coefficient of the VNA port in accordance with embodiments of the present disclosure.
FIG. 6 is a block diagram of VNA ports, antennas, and a multiple state calibration network in accordance with embodiments of the present disclosure.
FIG. 7 is a block diagram of a scattering S-parameters model of an antenna of the MI system in accordance with embodiments of the present disclosure.
FIG. 8 is a block diagram of a port error box and antenna error model in accordance with embodiments of the present disclosure.
FIG. 9 is a block diagram of the VNA ports and the antennas configured to measure the transmission path coefficients of a VNA port to all remaining VNA ports in accordance with embodiments of the present disclosure.
FIG. 10 is a block diagram of the VNA ports and the antennas of the MI system configured with the test subject to illustrate post calibration processing for correcting inactive antenna measurement errors in accordance with embodiments of the present disclosure.

### DETAILED DESCRIPTION

In the following detailed description, for purposes of explanation and not limitation, representative embodiments disclosing specific details are set forth in order to provide a thorough understanding of the present teachings. However, it will be apparent to one having ordinary skill in the art having had the benefit of the present disclosure that other embodiments according to the present teachings that depart from the specific details disclosed herein remain within the scope of the appended claims. Moreover, descriptions of well-known apparatuses and methods may be omitted so as to not obscure the description of the example embodiments. Such methods and apparatuses are clearly within the scope of the present teachings.

The terminology used herein is for purposes of describing particular embodiments only, and is not intended to be limiting. The defined terms are in addition to the technical and scientific meanings of the defined terms as commonly understood and accepted in the technical field of the present teachings. As used in the specification and appended claims, the terms 'a', 'an' and 'the' include both singular and plural referents, unless the context clearly dictates otherwise. Thus, for example, 'a device' includes one device and plural devices.

The described embodiments relate generally to imaging systems. More specifically, the described embodiments relate to methods for improved calibration of MI systems having multiple VNA ports.

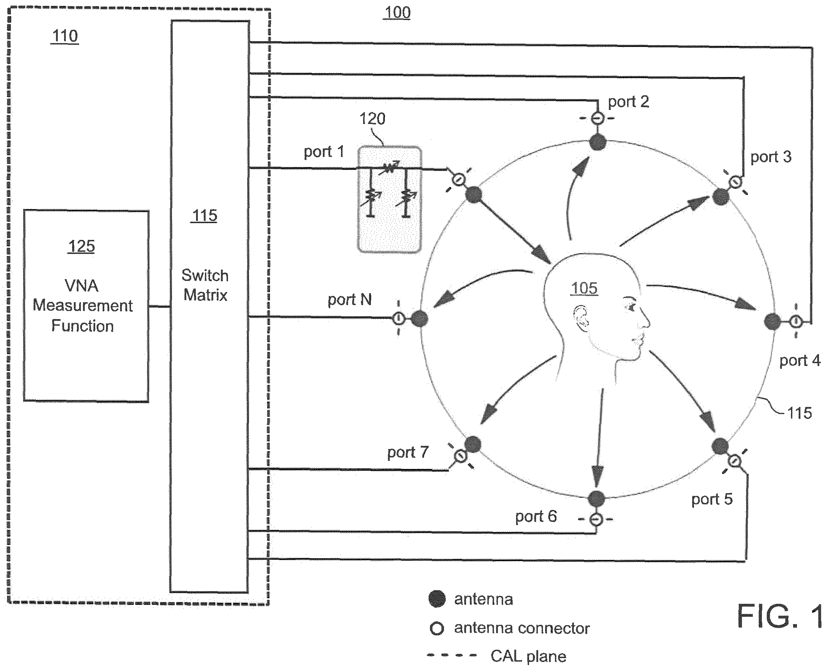

FIG. 1 is a block diagram of an example MI system 100 configured with a test subject 105 in accordance with embodiments of the present disclosure. The MI system 100 includes multiple VNA ports 110 that are operatively connected to multiple antennas 115. Each antenna has approximately equal transmission path and reflection parameters, and is directly coupled with a connector, wherein the connector defines a calibration (CAL) plane. A multiple state calibration network 120 is operatively connected between a first VNA port (port 1) of the VNA ports 110 (port 1-N) and a first antenna of the antennas 115. The multiple state calibration network 120 has predetermined parameters (i.e. known parameters) for each state. The VNA ports 110 include a VNA measurement function 125 operatively connected with a switch matrix 130. The VNA measurement function 125 is configured to measure transmission path and reflection coefficients.

In other embodiments, the VNA ports 110 may include at least three VNA ports. In other embodiments, the VNA ports 110 may include at least 100 VNA ports. In other embodiments, the VNA measurement function 125 may be configured to measure transmission path and reflection coefficients for a frequency that is in a frequency range between 500 megahertz (MHz) and 12 gigahertz (GHz). The multiple state calibration network may also be a multiple state two port passive network and the set of predetermined parameters comprise a set of two port S-parameter matrices. In other embodiments, the VNA ports 110 may include additional VNA measurement functions 125.

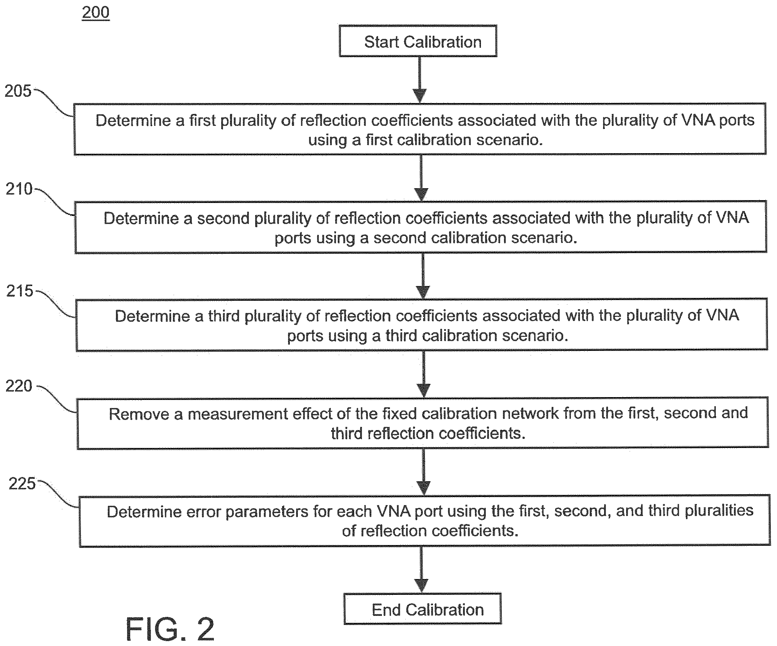

FIG. 2 is a flow chart of an example method 200 of calibrating the MI system 100 of FIG. 1 in accordance with a representative embodiment of the present disclosure. The method includes determining 205 a first set of reflection coefficients associated with the VNA ports 110 using a first calibration scenario. In the first calibration scenario, the MI system 100 is configured without the test subject 105. Only air is present in an imaging volume in the first calibration scenario.

The method 200 further includes determining 210 a second set of reflection coefficients associated with the VNA ports 110 using a second calibration scenario. In the second calibration scenario, the MI system 100 is configured with a first homogenous phantom positioned approximately equidistant from each antenna 115. A relative permittivity of the first homogenous phantom may be at least 2.0.

Step 210 may include prompting an operator of the MI system 100 to position the first homogenous phantom.

The method 200 further includes a step 215 of determining a third set of reflection coefficients associated with the VNA ports 110 using a third calibration scenario. In the third calibration scenario, the MI system 100 is configured with a second homogenous phantom positioned approximately equidistant from each antenna of the antennas 115. A relative permittivity of the second homogenous phantom may be at least two times greater than the relative permittivity of the first homogenous phantom. Step 215 may include prompting an operator of the MI system 100 to remove the first homogenous phantom and position the second homogenous phantom.

The method 200 further includes a step 220 of removing a measurement effect of the multiple state calibration network 120 from the first, second and third pluralities of reflection coefficients. The measurement effect may be removed by the process of de-embedding the fixed calibration network 120. For example, a de-embedding process as described in Agilent Application Note 1361-1 titled *De-embedding and Embedding S-Parameter Networks Using a Yector Network Analyzer* may be used, the subject matter of which is hereby incorporated by reference.

The method 200 further includes the step 225 of determining error parameters for each VNA port using the first, second, and third pluralities of reflection coefficients. The error parameters may include determining error box coefficients for each VNA port. The error box coefficients may each include a two port S-parameter matrix. The error box coefficients allow a calibration (CAL) plane at the output of the VNA measurement function 125 to be transferred to the associated antenna connector. After proper calibration, each VNA port will measure an approximately equal reflection coefficient within a calibration scenario (e.g. first, second, or third calibration scenarios).

In other embodiments, the method 200 may further include the step (not shown in FIG. 2) of determining transmission path coefficients for each VNA port. The transmission path coefficients can include transmission path coefficients associated with each of the remaining VNA ports 105. The transmission path coefficients may be determined using an "unknown thru" method. For example, the "unknown thru" method may be used as described in IEEE Microwave and Guided Wave Letters (December 1992 Volume:2, Issue: 12, pages 505-507 ) titled *Two-port network analyzer calibration using an unknown 'thru',* the subject matter of which is hereby incorporated by reference

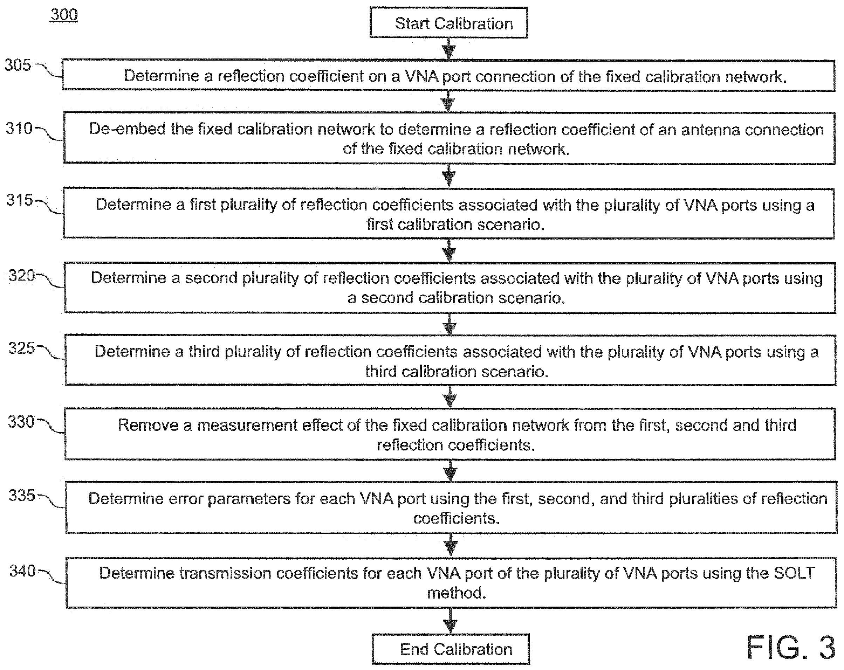

FIG. 3 is a flow chart illustrating another method 300 of calibrating the MI system 100 of FIG. 1 in accordance with a representative embodiment of the present disclosure. The method 300 includes a step 305 of determining a set of reflection coefficients on the first VNA port (port 1) connection of the multiple state calibration network 120. Step 305 may use the first calibration scenario as described in step 205 of FIG. 2.

The method 300 further includes the step 310 of de-embedding the multiple state calibration network 120 to determine a reflection coefficient of an antenna connection of the multiple state calibration network 120. The de-embedding process as described in Agilent Application Note 1361-1 titled *De-embedding and Embedding S-Parameter Networks Using a Vector Network Analyzer* may be used.

The method 300 further includes the step 315 of determining a first set of reflection coefficients associated with the VNA ports 110 using a first calibration scenario. Step 315 may use the first calibration scenario as described in step 205 of FIG. 2.

The method 300 further includes the step 320 of determining a second set of reflection coefficients associated with the VNA ports using a second calibration scenario. Step 320 may use the second calibration scenario as described in step 210 of FIG. 2.

The method 300 further includes the step 325 of determining a third set of reflection coefficients associated with the VNA ports using a third calibration scenario. Step 325 may use the third calibration scenario as described in step 215 of FIG. 2.

The method 300 further includes the step 330 of removing a measurement effect of the multiple state calibration network 120 from the first, second and third reflection coefficients. The measurement effect may be removed by the process of de-embedding the multiple state calibration network 120 as described in step 310.

The method 300 further includes determining 335 error parameters for each VNA port using the first, second, and third pluralities of reflection coefficients. The error parameters may include determining error box coefficients for each VNA port as described in step 225 of FIG. 2. After proper calibration, each VNA port will measure an approximately equal reflection coefficient within a calibration scenario (e.g. first, second, or third calibration scenarios).

The method 300 may further include determining 340 transmission path coefficients for each VNA port using a short-open-load-reciprocal thru ((SOLR) method. Transmission path coefficients may be determined using an "unknown thru" method. For example, the SOLR method as described in IEEE Microwave and Guided Wave Letters (December 1992 Volume:2, Issue: 12, pages 505-507) titled *Two-port network analyzer calibration using an unknown 'thru',* incorporated herein by reference, may be used.

The method 200 of FIG. 2 and the method 300 of FIG. 3 each produce repeatability of calibrated reflection and transmission measurements having a difference of less than 1 decibel (dB) and 1 degree.

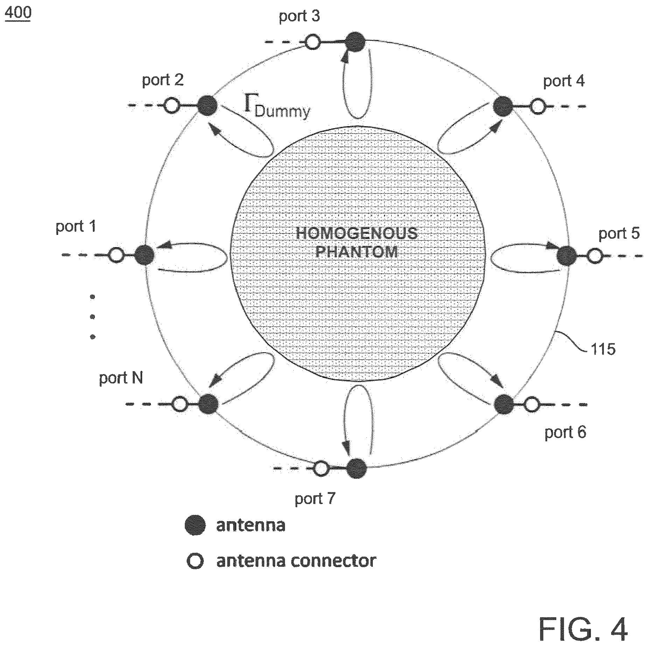

FIG. 4 is a block diagram of VNA ports 110 and antennas 115 configured in a calibration scenario with a homogenous phantom in accordance with embodiments of the present disclosure. As shown, a homogenous phantom is positioned approximately equidistant from each antenna 115. Exact permittivity of the homogeneous phantom is not required. However, the homogeneous phantom needs to be electrically homogenous throughout.

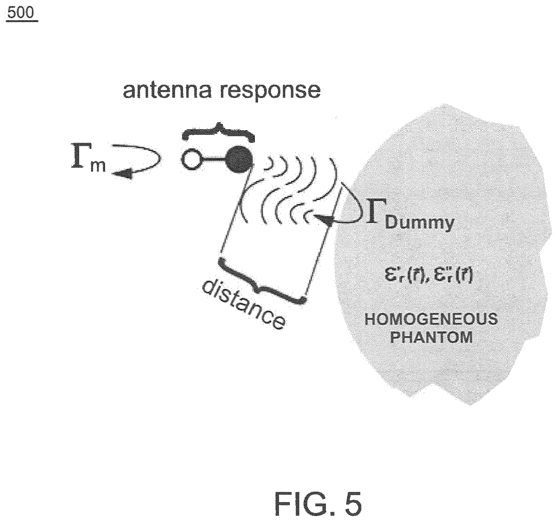

FIG. 5 is an enlarged diagram of an example VNA port and an antenna of the MI system 100 configured for measuring a reflection coefficient of the VNA port in accordance with embodiments of the present disclosure. The VNA port and the antenna may be representative of any VNA port and operatively connected antenna of the VNA ports 110 and the antennas 115.

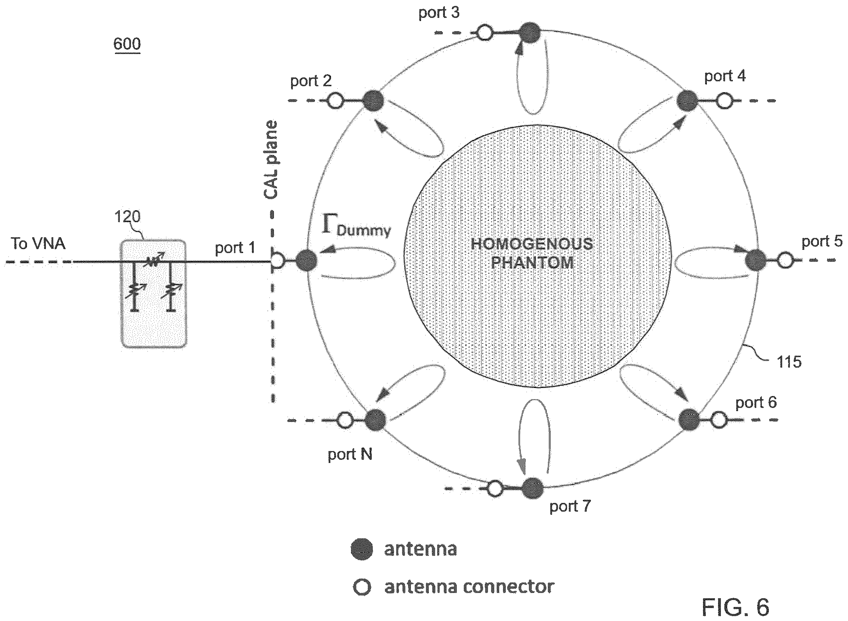

FIG. 6 is a block diagram of VNA ports 110, antennas 115, and a multiple state calibration network 120 in accordance with embodiments of the present disclosure. The CAL plane is shown as described in step 225 of FIG. 2 and step 335 of FIG. 3.

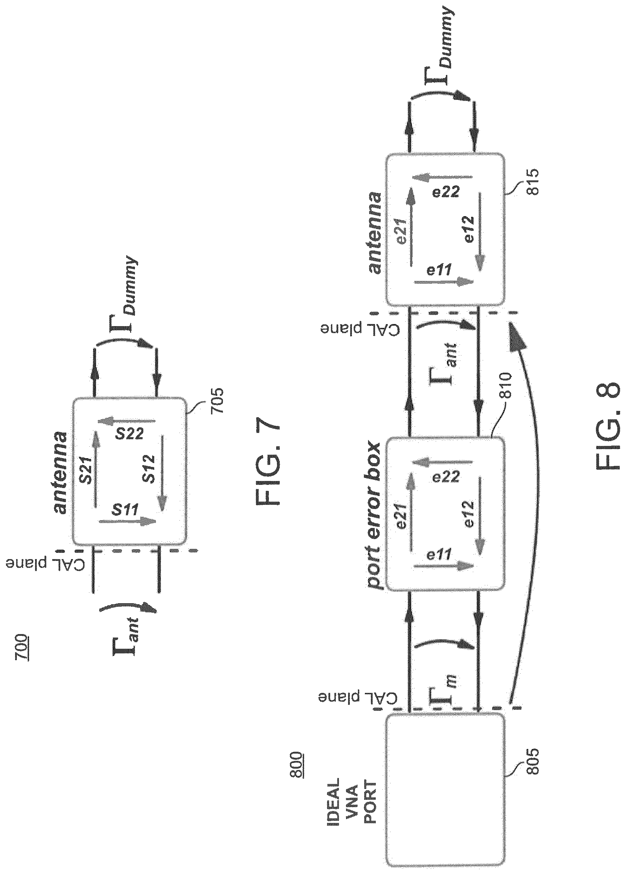

FIG. 7 is a block diagram of an example scattering S-parameters model 705 of an antenna 115 in accordance with embodiments of the present disclosure. For each antenna 115, receive and transmit math is fully reciprocal such that S₁₂ is approximately equal to S₂₁.

FIG. 8 is a block diagram of an example ideal VNA port 805, a port error box 810, and antenna error model 815 in accordance with embodiments of the present disclosure. Error box coefficients and antenna error coefficients (e11, e21, e12, and e22) may be S-parameters. In other embodiments, error box coefficients and antenna error coefficients (e11, e21, e12, and e22) may be transmission T-parameters.

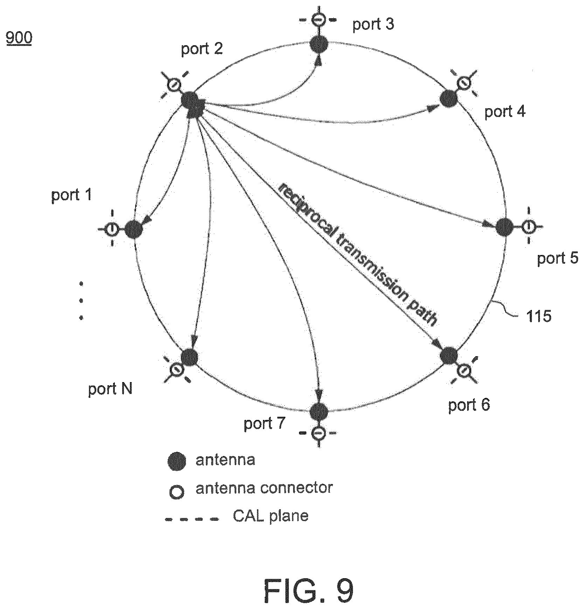

FIG. 9 is a block diagram of VNA ports 110 and antennas 115 configured to measure the transmission path coefficients of a VNA port to all remaining VNA ports as described in step 340 of FIG. 3. In some embodiments, to avoid noise when imaging, a path attenuation between any antenna combination of antennas 115 is less than 50 dB. In other embodiments, the path attenuation is less than 40 dB.

In other embodiments, post calibration processing of the MI system 100 may be required when imaging due to measurement error associated with inactive antennas among antennas 110. The termination of these inactive antennas may affect measured S-parameters of transmission path coefficients.

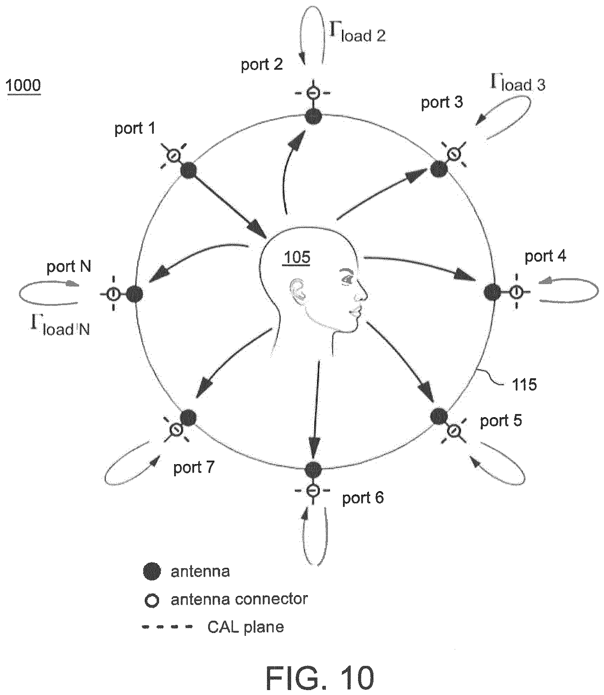

FIG. 10 is a block diagram of VNA ports 110 and antennas 110 of the MI system 100 configured with the test subject 105 to illustrate post calibration processing in accordance with embodiments of the present disclosure. Post calibration processing may use the first set of reflection coefficients from step 315 of FIG. 2 and the first plurality of transmission path coefficients of step 340 of FIG. 3 to de-embed the inactive antennas. A post calibration processing method may be used as described in IEEE Transactions on Microwave Theory and Techniques (May 1983 Volume: 31, Issue: 5, pages 407- 412) titled *Techniques for Correcting Scattering Parameter Data of an Imperfectly Terminated Multiport When Measured with a Two-Port Network Analyzer,* the subject matter of which is hereby incorporated by reference. However, when transmission path loss is low within the MI system 100, the measurement error associated the inactive antennas is greatly attenuated the post calibration process may not be needed.

The descriptions of the various embodiments of the present disclosure have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein. Therefore, the embodiments disclosed should not be limited to any single embodiment, but rather should be construed in breadth and scope in accordance with the appended claims.

## Claims

1. A method for calibrating a microwave imaging (MI) system, the MI system comprising a plurality of vector network analyzer (VNA) ports operatively connected to a plurality of antennas, wherein a multiple state calibration network having predetermined parameters is operatively connected between a first VNA port of the plurality of VNA ports and a first antenna of the plurality of antennas, the method comprising:
determining a first plurality of reflection coefficients associated with the plurality of VNA ports using a first calibration scenario;
determining a second plurality of reflection coefficients associated with the plurality of VNA ports using a second calibration scenario;
determining a third plurality of reflection coefficients associated with the plurality of VNA ports using a third calibration scenario;
removing a measurement effect of the multiple state calibration network from the first, second and third reflection coefficients; and
determining error parameters for each VNA port using the first, second, and third pluralities of reflection coefficients.

2. The method of claim 1, wherein the plurality of VNA ports comprises at least three VNA ports.

3. The method of claim 2, wherein the second calibration scenario comprises positioning a first homogenous phantom approximately equidistant from each antenna of the plurality of antennas.

4. The method of claim 3, wherein the third calibration scenario comprises positioning a second homogenous phantom approximately equidistant from each antenna of the plurality of antennas.

5. The method of claim 4, wherein a relative permittivity of the first homogenous phantom is at least 2.0.

6. The method of claim 5, wherein a relative permittivity of the second homogenous phantom is at least two times greater than the relative permittivity of the first homogenous phantom.

7. The method of claim 6, further comprising prompting an operator of the MI system to position the first and second homogenous phantoms.

8. The method of claim 7, wherein the error parameters for each VNA port of the plurality of VNA ports each comprise a two port S-parameter matrix.

9. The method of claim 8, wherein the multiple state calibration network is a multiple state two port passive network and the predetermined parameters comprise a set of two port S-parameter matrices.

10. The method of claim 1, further comprising determining a plurality of transmission path coefficients for each VNA port of the plurality of VNA ports, wherein the transmission coefficients comprise transmission path coefficients associated with each of the remaining VNA ports.

11. A method for calibrating a microwave imaging (MI) system, the MI system comprising a plurality of vector network analyzer (VNA) ports operatively connected to a plurality of antennas, wherein a multiple state calibration network having predetermined parameters is operatively connected between a first VNA port of the plurality of VNA ports and a first antenna of the plurality of antennas, the method comprising:
determining a set of reflection coefficient on a VNA port connection of the multiple state calibration network;
de-embedding the multiple state calibration network to determine a reflection coefficient of an antenna connection of the multiple state calibration network;
determining a first plurality of reflection coefficients associated with the plurality of VNA ports using a first calibration scenario;
determining a second plurality of reflection coefficients associated with the plurality of VNA ports using a second calibration scenario;
determining a third plurality of reflection coefficients associated with the plurality of VNA ports using a third calibration scenario;
removing a measurement effect of the multiple state calibration network from the first, second and third reflection coefficients; and
determining error parameters for each VNA port using the first, second, and third pluralities of reflection coefficients.

12. The method of claim 11, further comprising determining transmission path coefficients for each VNA port of the plurality of VNA ports using a short-open-load-reciprocal thru (SOLR) method.

13. The method of claim 12, wherein the plurality of VNA ports comprises at least three VNA ports.

14. The method of claim 13, wherein the second calibration scenario comprises positioning a first homogenous phantom approximately equidistant from each antenna of the plurality of antennas.

15. The method of claim 14, wherein the third calibration scenario comprises positioning a second homogenous phantom approximately equidistant from each antenna of the plurality of antennas.

16. The method of claim 15, wherein a relative permittivity of the first homogenous phantom is at least 2.0.

17. The method of claim 16, wherein a relative permittivity of the second homogenous phantom is at least two times greater than the relative permittivity of the first homogenous phantom.

18. The method of claim 17, further comprising prompting an operator of the MI system to position the first and second homogenous phantoms.

19. The method of claim 18, wherein the error parameters for each VNA port of the plurality of VNA ports each comprise a two port S-parameter matrix.

20. The method of claim 19, wherein the calibration network is a multiple state two port passive network and the predetermined parameters comprise a set of two port S-parameter matrices.
